# EUROPÄISCHE PATENTSCHRIFT

(11) **EP 1 001 722 B1**
(45) Veröffentlichungstag und Bekanntmachung des Hinweises auf die Patenterteilung: **01.10.2003**
(21) Anmeldenummer: 97932696.4
(22) Anmeldetag: 06.08.1997
(51) Int. Cl.: A61F 2/44

(54) **SPREIZBARES ZWISCHENWIRBELIMPLANTAT**
INTERVERTEBRAL IMPLANT WHEREOF THE PARTS CAN BE SPACED
IMPLANT INTERVERTEBRAL DONT LES PARTIES PEUVENT ETRE ECARTEES

(43) Veröffentlichungstag der Anmeldung: 24.05.2000
(73) Patentinhaber: SYNTHES AG Chur, 7002 Chur (CH)
(72) Erfinder: AEBI, Max, Montreal, Quebec H2V 4P1 (CA); KNOTE, Inga, CH-2540 Grenchen (CH); BENOIT, Alfred, CH-2543 Lengnau (CH)
(74) Vertreter: Lusuardi, Werther Giovanni, Dr.
(86) Internationale Anmeldenummer: CH9700293
(87) Internationale Veröffentlichungsnummer: WO99007312

(56) Entgegenhaltungen:
- EP-A- 0 664 994
- DE-C- 4 416 605
- US-A- 5 653 763

## Beschreibung

Die Erfindung betrifft ein Zwischenwirbelimplantat gemäss dem Oberbegriff des Patentanspruchs 1.

Aus der EP-A1 0 664 994 ist ein solches Zwischenwirbelimplantat bekannt. Die Nachteile dieser Anordnung bestehen darin, dass das Implantat einstückig mit der Spreizvorrichtung ausgebildet ist, so dass es nur als Ganzes in den Zwischenwirbelraum einführbar ist, wobei die Einführung durch Einschlagen des Implantats erfolgen muss. Des weiteren lässt sich das bekannte Zwischenwirbelimplantat nur stufenlos aufspreizen, was dazu führt, dass lediglich eine ungenaue präoperative Planung des Aufspreizwinkels möglich ist.

Hier will die Erfindung Abhilfe schaffen. Der Erfindung liegt das Problem zugrunde, ein Zwischenwirbelimplantat zu schaffen, welches in einer ersten Phase - ohne Spreizvorrichtung - in den Zwischenwirbelraum eingeschraubt werden kann und erst in einer zweiten Phase mittels einer in das Implantat einführbaren Spreizvorrichtung im Zwischenwirbelraum verspreizt wird. Diese Verspreizung soll stufenlos oder kontrolliert erfolgen können, so dass eine präoperative Planung des Aufspreizwinkels ermöglicht wird.

Die Erfindung löst die gestellte Aufgabe mit einem Zwischenwirbelimplantat, welches die Merkmale des Anspruchs 1 aufweist.

Der Führungszylinder des Implantats ist entweder mit einem Innengewinde oder mit einem Aussengewinde (oder auch mit beidem) versehen. Das Innengewinde erlaubt eine Aufspreizung des Implantats mittels einer Spreizvorrichtung, welche einen Schaft mit einem zum Innengewinde korrespondierenden Gewinde besitzt. Das Aussengewinde auf dem Führungszylinder erlaubt aber auch eine Aufspreizung des Implantats mittels einer Spreizvorrichtung, welche einen Hohlzylinderteil mit einem zum Aussengewinde korrespondierenden Gewinde besitzt. Die zweite Variante erlaubt wegen der grösseren Durchmesser die Übertragung grösserer Zugkräfte.

Die Ausführung des Führungszylinders mit einem Innen- und einem Aussengewinde hat den zusätzlichen Vorteil, dass eine geführte Operationstechnik möglich ist. Ein im Innengewinde fixierter Führungsstift kann während der gesamten Operation als Führungshilfe benutzt werden, über welche die anderen Instrumente geschoben werden können. Die Operation findet dabei im wesentlichen in drei Schritten statt. Einschrauben des Implantats mit Hilfe einer Gewindehülse, Auffüllung des Implantats mit Knochenspänen, Verspreizung des Implantats im Zwischenwirbelraum gegen die Endplatten. Denkbar ist noch ein zusätzlicher Schritt der darin besteht das Knochenwachstum stimulierende Materialien in flüssiger Form oder als Gel in den Zwischenwirbelraum einzuspritzen. Zu diesem Zweck können perforierte Einschraub- oder Knochenspan-Insertions-Instrumente verwendet werden.

Eine weitere Ausbildung des Zwischenwirbelimplantats besteht darin, dass die Aussenseiten der Schenkel mit im wesentlich parallel zum Führungszylinder verlaufenden Führungsrippen versehen sind, was die Einführung des Implantats in den Zwischenwirbelraum erleichtert.
Die Schenkel können auch mit Perforationen versehen sein, was das Einwachsen des Knochens begünstigt. Zum gleichen Zweck können auch je zwei Schenkel in den beiden Ebenen des U-förmigen Stützkörpers vorgesehen sein, wobei die Schenkel an den Enden des Stegs angebracht sind, so dass zwischen den beiden Schenkeln in jeder Ebene ein freier Raum besteht, in welchen der Knochen einwachsen kann.
Der Steg des Implantats ist vorzugsweise symmetrisch zu den Schenkeln angeordnet.

Eine bevorzugte Weiterbildung besteht darin, dass die Innenseiten der Schenkel des Implantats mit Rillen versehen sind, welche quer zum Führungszylinder verlaufen. Dies hat den zusätzlichen Vorteil, dass beim Aufspreizen des Zwischenwirbelimplantats das Einstellen eines definierten (präoperative geplanten) Winkels möglich wird.

Eine weitere Ausbildung des Zwischenwirbelimplantats besteht darin, dass seine Schenkel an ihrem vorderen freien Ende eine nach Innen gerichtete Lippe aufweisen; sie verhindert das Herausrutschen der in das Implantat eingefüllten Knochenspäne.

Die durch die Erfindung erreichten Vorteile sind im wesentlichen darin zu sehen, dass dank des erfindungsgemässen Zwischenwirbelimplantats:
- der natürliche Lordoseradius der Wirbelsäule patient engerecht wiederhergestellt werden kann und dass dieser Winkel präoperativ bestimmt werden kann. Dadurch ergeben sich für das Implantat optimale Voraussetzungen für dessen Einwachsen;
- eine atraumatische Insertionstechnik ermöglicht wird, indem das Implantat sanft und kontrolliert eingeschraubt werden kann, anstelle eines Einschlagvorgangs; und
- eine vereinfachte (auch minimalinvasive ) Operationstechnik ermöglicht wird: 3-Schritt-Operationstechnik über einen Führungsstift geführt.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Darstellung des erfindungsgemässen Zwischenwirbelimplantats;
Fig. 2 eine Hülse mit Gewinde für das Zwischenwirbelimplantat nach Fig. 1;
Fig. 3 eine perspektivische Darstellung einer Spreizvorrichtung mit Mutter für das Zwischenwirbelimplantat nach Fig. 1;
Fig. 4 eine perspektivische Darstellung einer modifizierten Spreizvorrichtung mit Schraube für das Zwischenwirbelimplantat nach Fig. 1;
Fig. 5 einen Schnitt durch das erfindungsgemässe Zwischenwirbelimplantat mit eingesetzter Hülse mit Gewinde;
Fig. 6 eine perspektivische Ansicht einer modifizierten Hülse mit Gewinde und hohlzylindrischem Schaft;
Fig. 7 einen Schnitt durch das erfindungsgemässe Zwischenwirbelimplantat mit eingesetzter Hülse nach Fig. 6;
Fig. 8 einen Schnitt durch das erfindungsgemässe Zwischenwirbelimplantat mit einem teilweise darin eingeführten Knochenspan-Impaktor zur Auffüllung des Stützkörpers mit Knochenspänen;
Fig. 9 eine perspektivische Teilansicht des Knochenspan-Impaktors nach Fig. 8;
Fig. 10 einen Schnitt durch ein erfindungsgemässes Zwischenwirbelimplantat nach Fig. 1 mit einer eingesetzten Spreizvorrichtung nach Fig. 3, welche mittels eines über den Führungsstift geschobenen Einführungsinstrumentes in den Stützkörper eingeschraubt wird;
Fig. 11 eine perspektivische Teilansicht des Einführungsinstrumentes nach Fig. 10;
Fig. 12 einen Längsschnitt durch ein modifiziertes erfindungsgemässes Zwischenwirbelimplantat (mit verkürztem Führungszylinder) und darin eingeschraubter Spreizvorrichtung nach Fig. 4; und
Fig. 13 eine Vorderansicht des Zwischenwirbelimplantats nach Fig. 12 mit eingeschraubter Spreizvorrichtung.

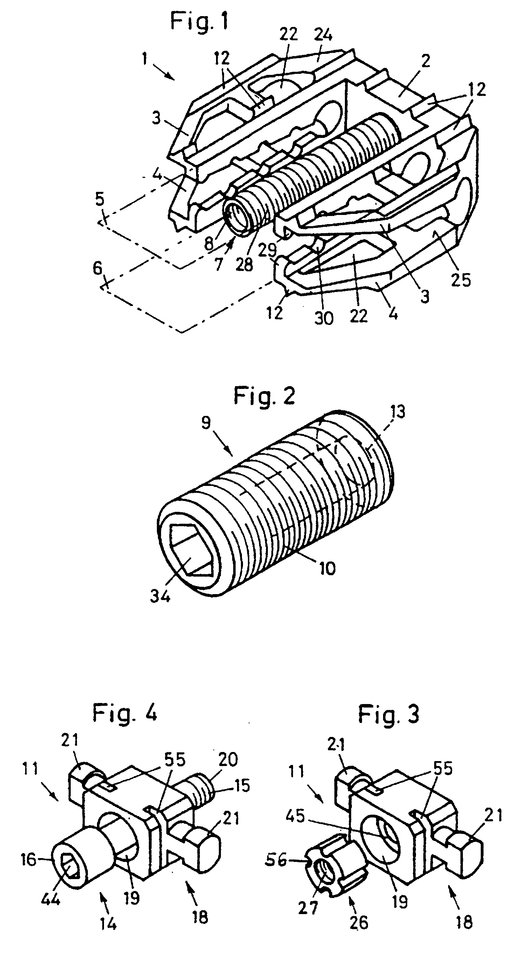

Das in Fig. 1 dargestellte Zwischenwirbelimplantat besitzt die Form eines Stützkörpers 1, welcher einen zentralen Steg 2 und einstückig am Steg 2 befestigte Schenkel 3,4 umfasst. Die Schenkel 3,4 sind in zwei im wesentlichen parallel zueinander verlaufenden, durch den Steg 2 beabstandete Ebenen 5,6 und symmetrisch zu diesem angeordnet, wobei die Ebenen 5,6 als Stützflächen an benachbarte Wirbelkörper anlegbar sind.

Der Steg 2 weist einen im wesentlichen parallel zu den Schenkeln 3,4 und zwischen diesen verlaufenden, hohlen Führungszylinder 7 mit einem Innengewinde 8 und einem Aussengewinde 28 auf. Die Innenseiten 25 der Schenkel 3,4 sind mit Rillen 30 versehen, welche quer zum Führungszylinder 7 verlaufen. Die Aussenseiten 24 der Schenkel 3,4 sind mit im wesentlich parallel zum Führungszylinder 7 verlaufenden Führungsrippen 12 versehen. Die Schenkel 3,4 sind im übrigen mit Perforationen 22 versehen und besitzen an ihrem vorderen freien Ende eine nach Innen gerichtete Lippe 29.

Je zwei Schenkel 3;4 sind in den beiden Ebenen 5,6 des U-förmigen Stützkörpers 1 vorgesehen, wobei die Schenkel 3,4 an den Enden des Stegs 2 angebracht sind, so dass zwischen den beiden Schenkeln in jeder Ebene 5,6 ein freier Raum 23 besteht.

In Fig. 2 ist eine Hülse 9 mit Aussengewinde 10, Hohlraum 13 und Sechskantvertiefung 34 dargestellt, welche auf den Führungszylinder 7 des Stützkörpers 1 geschoben werden kann. Sie hat vorzugsweise einen Durchmesser, der leicht grösser ist als die Höhe des Stützkörpers 1 (Abstand der Ebenen 5 und 6). Die in den Stützkörper 1 eingeführte Hülse 9 kann mittels eines geeigneten, in die Sechskantvertiefung 34 eingeführten Instrumentes um den Führungszylinder 7 rotiert werden und dient zur Einführung des Stützkörpers 1 in den Zwischenwirbelraum. Da die Hülse 9 etwas über die Aussenseiten 24 der Schenkel 3,4 hervorragt, greift ein Teil ihres Aussengewindes 10 in das Knochenmaterial der benachbarten Wirbelkörper, so dass damit der Stützkörpers 1 ohne Mühe in den Zwischenwirbelraum eingedreht werden kann. Nach erfolgter Einführung des Stützkörpers 1 in den Zwischenwirbelraum kann dann die Hülse 9 ebenso leicht wieder über den Führungszylinder 7 zurück- und ausgeschraubt werden.

In den Fig. 3 und 4 sind zwei Varianten für eine, den Stützkörper 1 expandierende Spreizvorrichtung 11 dargestellt, welche beide einen identischen Spreizkörper 18 umfassen. Der Spreizkörper 18 hat die Form eines Quaders mit einer zentralen Bohrung 19, an welchem seitlich zwei Nocken 21 angebracht sind, welche zwischen die Schenkel 3,4 einführbar sind. Der Spreizkörper 18 weist zudem zwei Seitenschlitze 55 auf, welche der Erfassung und Handhabung des Spreizkörpers 18 dienen.
Die erste - in Fig. 3 dargestellte - Variante der Spreizvorrichtung 11 umfasst im weiteren eine hohlzylinderförmige Mutter 26 mit Innengewinde 27, welche mit dem Aussengewinde 28 des Führungszylinders 7 korrespondiert. Die Bohrung 19 ist derart ausgestaltet, dass die Mutter 26 bis zum Anschlag 45 darin einführbar ist.
Die zweite - in Fig. 4 dargestellte - Variante der Spreizvorrichtung 11 umfasst (statt der Mutter 26) eine Schraube 14 mit einem, einen Innensechskant 44 aufweisenden Kopf 16 und einem, ein Aussengewinde 15 tragenden Schaft 20. Das Aussengewinde 15 korrespondiert dabei mit dem Innengewinde 8 des Führungszylinders 7. Der Kopf 16 der Schraube 14 kommt - analog zur Mutter 26 (Fig. 3) - am Anschlag 45 im Inneren der zentralen Bohrung 19 zum Anliegen.

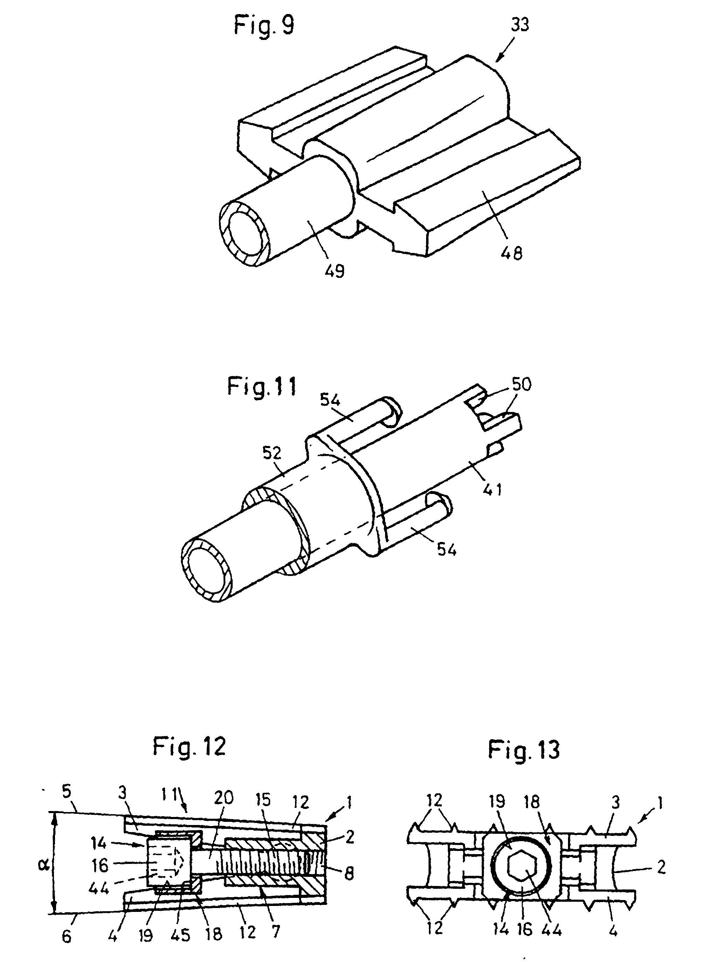

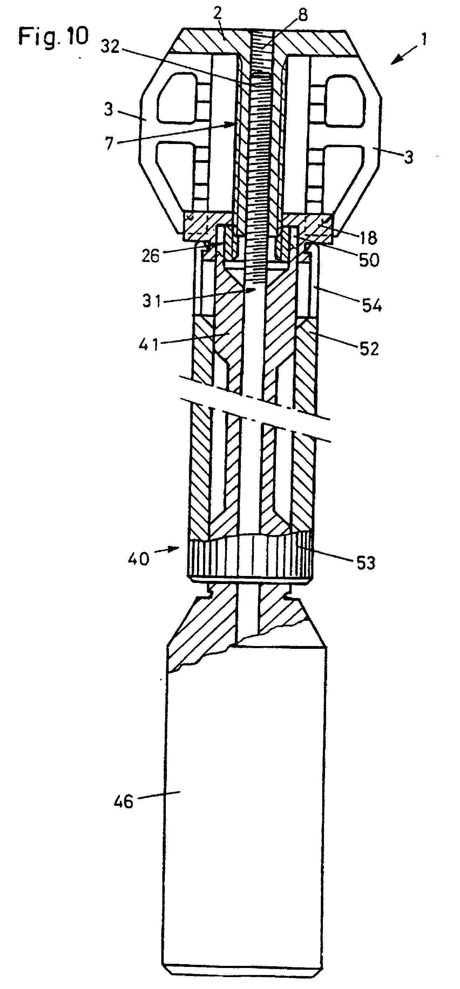

Bei Verwendung der zweiten, in Fig. 4 dargestellten Variante der Spreizvorrichtung 11 muss der Führungszylinder 7 verkürzt werden, so wie dies in Fig. 12 dargestellt ist, d.h. etwa halb so lang sein, wie in Fig. 1 dargestellt.

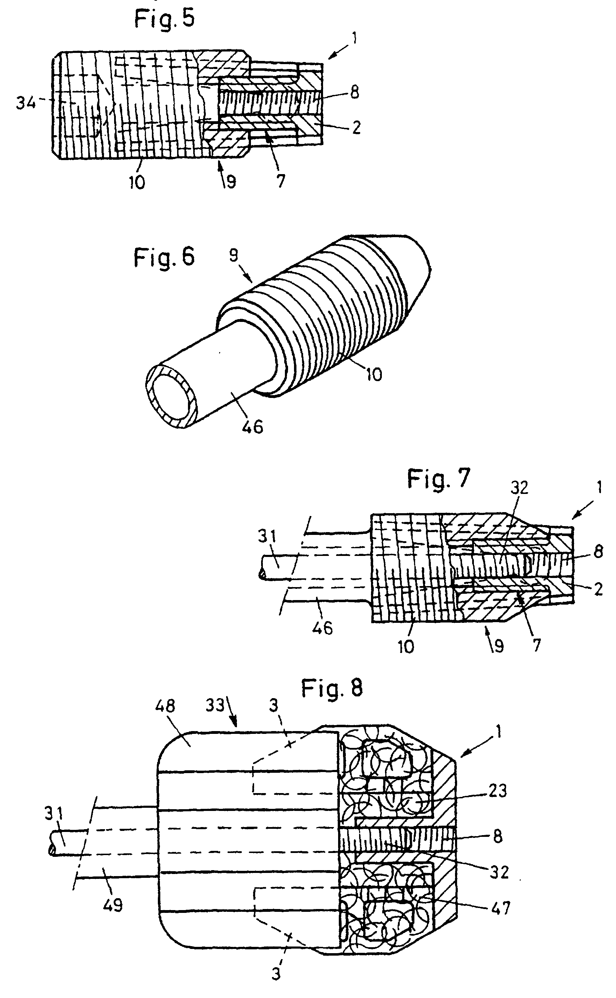

Anhand der Figuren 5 - 13 wird nun die Operationstechnik des erfindungsgemässen Zwischenwirbelimplantats näher beschrieben.

Nach erfolgter Entfernung von genügend Bandscheibenmaterial wird der U-förmige Stützkörper 1 zwischen die betroffenen Wirbel eingeführt. Zu diesem Zweck wird - wie in Fig. 5 dargestellt-über dessen Führungszylinder 7 die Hülse 9 mit Aussengewinde 10 aufgeschoben. Der U-förmige Stützkörper 1 mit der aufgeschobenen Hülse 9 wird leicht in den aufbereiteten Zwischenwirbelraum eingeklopft, bis der erste Gang des Aussengewindes 10 in das Knochenmaterial greift. Dann wird der U-förmige Stützkörper 1 durch weiteres Eindrehen der Hülse 9 auf die gewünschte Tiefe in den Zwischenwirbelraum eingeschraubt. Das Eindrehen der Hülse 9 kann dabei durch ein in den Innensechskant 34 eingeführtes Eindrehinstrument erfolgen.

Bei einer in den Fig. 6 und 7 dargestellten Variante ist die Hülse 9 mit Aussengewinde 10 an einem hohlzylindrischen Schaft 46 befestigt und stellt einen Teil eines Einführungsinstrumentariums dar. Um das Einführungsinstrumentarium axial besser führen zu können, kann vorgängig ein an seinem vorderen Ende mit einem Aussengewinde 32 (Fig. 10) versehener Führungsstift 31 (Fig. 7) in das Innengewinde 8 des Führungszylinders 7 des Stützkörpers 1 eingeschraubt werden, wo er während der gesamten Operationszeit verbleiben kann. Die Hülse 9 gemäss Fig. 6 kann dann einfach mit ihrem hohlzylindrischen Schaft 46 über den Führungsstift 31 auf den Führungszylinder 7 geschoben und dort rotiert werden.

Die auf den Aussenseiten 24 der Schenkel 3,4 des Stützkörpers 1 verlaufenden Führungsrippen 12 verhindern, dass der Stützkörper 1 während des Einschraubvorgangs von der gewünschten Richtung abweicht.

Die Hülse 9 wird nach Abschluss des Einschraubvorgangs wieder aus dem Stützkörper 1 herausgeschraubt. Da zwischen der Oberfläche des Führungszylinders 7 und der Innenfläche der Hülse 9 praktisch keine Reibung besteht, bleibt der Stützkörper 1 während des Herausschraubens der Hülse 9 unverändert an seinem Ort.

Nun schliesst sich fakultativ - wie in Fig. 8 dargestellt - die Einführung von Knochenspänen 47 in den freien Raum 23 des Stützkörpers 1 an. Zu diesem Zweck werden - mittels eines Knochenspan-Impaktors 33 - Knochenspäne 47 in den freien Raum 23 eingeführt und komprimiert. Der in Fig. 8 dargestellte Knochenspan-Impaktor 33 besteht aus einem flachen Grundkörper 48, der - wie in Fig. 8 gezeigt - teilweise zwischen die Schenkel 3,4 einführbar und an einem hohlzylindrischen Schaft 49 befestigt ist. Auch der Knochenspan-Impaktor 33 kann über den Führungsstift 31 axial geführt werden.

Schliesslich erfolgt - wie in Fig. 10 dargestellt - die Einführung einer Spreizvorrichtung 11 zwischen die Ebenen 5,6 und den darin angeordneten Schenkeln 3,4 des Stützkörpers 1.
Die Spreizvorrichtung 11 gemäss Fig. 3 wird nun im zusammengesetzten Zustand (d.h. mit der in der Bohrung 19 eingesetzten Mutter 26), mittels des Einführungsinstrumentes 40, mit ihren beiden Nocken 21 zwischen die Schenkel 3,4 eingeführt.

Das vordere Ende des Einführungsinstrumentes 40 ist im Detail in Fig. 11 dargestellt. Die rotierbare Hülse 41 weist an ihrem vorderen Ende vier Antriebsnocken 50 für die Mutter 26 auf; zu diesem Zweck sind in der Mutter vier korrespondierende Längsnuten 56 eingelassen, so dass sich die Mutter 26 damit drehen lässt. Die rotierbare Hülse 41 ist in der Haltehülse 52 mit geriffeltem Ring 53 gelagert und weist an ihrem vorderen Ende zwei Haltestifte 54 auf, welche in die Seitenschlitze 55 (Fig. 3) der Nocken 21 einführbar sind, um damit den Spreizkörper 18 zu halten.

Bei Verwendung einer Spreizvorrichtung 11 nach Fig. 4, welche neben dem identischen Spreizkörper 18 eine Schraube 14 (statt der Mutter 26) umfasst, wird - wie in den Fig. 12 und 13 dargestellt - der Schaft 20 der Schraube 14 mit seinem Gewinde 15 in das Innengewinde 8 des hohlen Führungszylinders 7 eingeführt. Das Einschrauben der Schraube 14 in den Führungszylinder 7 bewirkt - wegen des Anschlags 45 des Schraubenkopfes 16 in der Bohrung 19 - eine axiale Verschiebung des Spreizkörpers 18 in Richtung des Führungszylinders 7, wobei die daran befestigten Nocken 21 in die Schenkel 3,4 eindringen und diese Aufspreizen. Durch kontinuierliches Eindrehen der Schraube 14 können die Schenkel 3,4 soweit aufgespreizt werden, dass ihre Ebenen 5,6 einen Winkel α von bis zu 10 - 12° einschliessen. Das Eindrehen der Schraube 14 kann mittels eines in die Sechskantöffnung 44 im Kopf 16 eingeführten Instrumentes erfolgen.

## Patentansprüche

1. Zwischenwirbelimplantat in Form eines Stützkörpers (1), welcher einen zentralen Steg (2) und einstückig am Steg (2) befestigte Schenkel (3,4) umfasst, wobei die Schenkel (3,4) in zwei im wesentlichen parallel zueinander verlaufenden, durch den Steg (2) beabstandeten Ebenen (5,6) angeordnet sind, welche als Stützflächen an benachbarte Wirbelkörper anlegbar sind und der Steg (2) einen im wesentlichen parallel zu den Schenkeln (3,4) und zwischen diesen verlaufenden Führungszylinder (7) mit einem Gewinde (8;28) aufweist, **dadurch gekennzeichnet, dass**
A) der Führungszylinder (7) drehfest am zentralen Steg (2) angeordnet; und
B) der Führungszylinder (7) an seinem dem Steg (2) abgewandten Ende hohlzylindrisch ausgebildet ist.

2. Zwischenwirbelimplantat nach Anspruch 1, **dadurch gekennzeichnet, dass** der hohle Führungszylinder (7) entweder ein Innengewinde (8) oder ein Aussengewinde (28) besitzt.

3. Zwischenwirbelimplantat nach Anspruch 1, **dadurch gekennzeichnet, dass** der hohle Führungszylinder (7) ein Innengewinde (8) und ein Aussengewinde (28) besitzt.

4. Zwischenwirbelimplantat nach einem der Ansprüche 1 bis 3, **dadurch gekennzeichnet, dass** die Innenseiten (25) der Schenkel (3,4) mit Rillen (30) versehen sind, welche quer zum Führungszylinder (7) verlaufen.

5. Zwischenwirbelimplantat nach einem der Ansprüche 1 bis 4, **dadurch gekennzeichnet, dass** die Aussenseiten (24) der Schenkel (3,4) mit im wesentlich parallel zum Führungszylinder (7) verlaufenden Führungsrippen (12) versehen sind.

6. Zwischenwirbelimplantat nach einem der Ansprüche 1 bis 4, **dadurch gekennzeichnet, dass** die Aussenseiten (24) der Schenkel (3,4) mit im wesentlich parallel zum Führungszylinder (7) verlaufenden Querrillen, insbesondere einem Fischgrätenprofil oder Sägezahnprofil versehen sind.

7. Zwischenwirbelimplantat nach einem der Ansprüche 1 bis 6, **dadurch gekennzeichnet, dass** die Schenkel (3,4) mit Perforationen (22) versehen sind.

8. Zwischenwirbelimplantat nach einem der Ansprüche 1 bis 7, **dadurch gekennzeichnet, dass** je zwei Schenkel (3;4) in den beiden Ebenen (5,6) des U-förmigen Stützkörpers (1) vorgesehen sind, wobei die Schenkel (3,4) an den Enden des Stegs (2) angebracht sind, so dass zwischen den beiden Schenkeln (3,4) in jeder Ebene (5,6) ein freier Raum (23) besteht.

9. Zwischenwirbelimplantat nach einem der Ansprüche 1 bis 8, **dadurch gekennzeichnet, dass** der Steg (2) symmetrisch zu den Schenkeln (3,4) angeordnet ist.

10. Zwischenwirbelimplantat nach einem der Ansprüche 1 bis 9, **dadurch gekennzeichnet, dass** die Schenkel (3;4) an ihrem vorderen freien Ende eine nach Innen gerichtete Lippe (29) aufweisen.

11. Zwischenwirbelimplantat nach einem der Ansprüche 1 bis 10, **dadurch gekennzeichnet, dass** es weiter einen im Innengewinde (8) des Führungszylinders (7) fixierbaren Führungsstift (31) umfasst.

12. Zwischenwirbelimplantat nach einem der Ansprüche 1 bis 11, **dadurch gekennzeichnet, dass** es weiter eine auf den Führungszylinder (7) aufschiebbare Hülse (9) mit Aussengewindeteil (10) umfasst.

13. Zwischenwirbelimplantat nach einem der Ansprüche 1 bis 12, **dadurch gekennzeichnet, dass** es weiter einen zwischen die Ebenen (5,6) und den darin angeordneten Schenkeln (3,4) einführbare Spreizvorrichtung (11) für die Schenkel (3,4) umfasst, wobei die Spreizvorrichtung eine Schraube (14) mit Schaft (20) mit einem zum Innengewinde (8) korrespondierenden Gewinde (15) aufweist.

14. Zwischenwirbelimplantat nach einem der Ansprüche 1 bis 12, **dadurch gekennzeichnet, dass** es weiter einen zwischen die Ebenen (5,6) und den darin angeordneten Schenkeln (3,4) einführbare Spreizvorrichtung (11) für die Schenkel (3,4) umfasst, wobei die Spreizvorrichtung eine hohlzylindrische Mutter (26) mit einem zum Aussengewinde (28) korrespondierenden Innengewinde (27) aufweist.

## Claims

1. An intervertebral implant in the form of a support unit (1) comprising a central yoke (2) and legs (3, 4) integral with said yoke, said legs (3, 4) being configured in two essentially mutually parallel planes (5, 6) apart by the yoke (2) and being abuttable as support surfaces against adjacent vertebrae, and the yoke (2) comprises a hollow guide cylinder (7) running essentially parallel to and between the legs (3, 4) and comprising at least one thread (8; 28),
**characterised in that**
A) the guide cylinder (7) is configured rotationally stable at the central yoke (2); and
B) the guide cylinder (7) is shaped hollow cylindrical at its end being remote of the yoke (2).

2. Intervertebral implant as claimed in claim 1, **characterized in that** the hollow guide cylinder (7) comprises either an inside thread (8) or an outside thread (28).

3. Intervertebral implant as claimed in claim 1, **characterized in that** the hollow guide cylinder (7) comprises an inside thread (8) and an outside thread (28).

4. Intervertebral implant as claimed in one of claims 1 through 3, **characterized in that** the inside surfaces (25) of the legs (3, 4) are fitted with grooves (30) running transversely to the guide cylinder (7).

5. Intervertebral implant as claimed in one of claims 1 through 4, **characterized in that** the outside surfaces (24) of the legs (3, 4) are fitted with guide ribs (12) running substantially parallel to the guide cylinder (7).

6. Intervertebral implant as claimed in one of claims 1 through 4, **characterized in that** the outside surfaces (24) of the legs (3, 4) are fitted with transverse grooves, in particular evincing a herringbone or a serrate contour, running substantially parallel to the guide cylinder (7).

7. Intervertebral implant as claimed in one of claims 1 through 6, **characterized in that** the legs (3, 4) comprise perforations (22).

8. Intervertebral implant as claimed in one of claims 1 through 7, **characterized in that** a pair of legs (3; 4) is present in each plane (5, 6) of the U-shaped support unit (1), the legs (3, 4) being mounted to the ends of the yoke (2) so as to subtend a free space (23) between the legs (3, 4) of each plane (5, 6).

9. Intervertebral implant as claimed in one of claims 1 through 8, **characterized in that** the yoke (2) is configured symmetrically relative to the legs (3, 4).

10. Intervertebral implant as claimed in one of claims 1 through 9, **characterized in that** the legs (3; 4) comprise an inwardly pointing lip (29) at their front free ends.

11. Intervertebral implant as claimed in one of claims 1 through 10, **characterized in that** it further comprises a guide spindle (31) affixable in the inside thread (8) of the guide cylinder (7).

12. Intervertebral implant as claimed in one of claims 1 through 11, **characterized in that** furthermore it comprises a bush (9) which is fitted with an outer threaded portion (10) and which can be slipped onto the guide cylinder (7).

13. Intervertebral implant as claimed in one of claims 1 through 12, **characterized in that** furthermore it comprises an expansion unit (11) for the legs (3, 4) that is insertable between the planes (5, 6) and the legs (3, 4) situated therein, said expansion unit comprising a screw (14) having a shank (20) with a thread (15) matching the inside thread (8).

14. Intervertebral implant as claimed in one of claims 1 through 12, **characterized in that** it furthermore comprises an expansion unit (11) for the legs (3, 4) that is insertable between the planes (5, 6) and the legs (3, 4) situated therein, said expansion unit comprising a hollow, cylindrical nut (26) fitted with an inside thread (27) matching the outside thread (28).

## Revendications

1. Implant intervertébral qui présente la forme d'un corps de soutien (1) qui comporte une branche centrale (2) et des branches (3, 4) fixées d'un seul tenant à la branche centrale (2), les branches (3, 4) étant disposées dans deux plans (5, 6) qui s'étendent essentiellement en parallèle l'un à l'autre et écartés par la branche centrale (2) et pouvant être placées comme surface de soutien sur des corps de vertèbres voisins, la branche centrale (2) présentant un cylindre de guidage (7) qui est doté de filets (8; 28)et qui est essentiellement parallèle aux branches (3, 4) et est situé entre ces dernières, **caractérisé en ce que**
A) le cylindre de guidage (7) est disposé fixe en rotation sur la branche centrale (2) et
B) le cylindre de guidage (7) est configuré en cylindre creux à son extrémité non tournée vers la branche centrale (2).

2. Implant intervertébral selon la revendication 1, **caractérisé en ce que** le cylindre de guidage creux (7) présente soit un filet intérieur (8) soit un filet extérieur (28).

3. Implant intervertébral selon la revendication 1, **caractérisé en ce que** le cylindre de guidage creux (7) présente un filet intérieur (8) et un filet extérieur (28).

4. Implant intervertébral selon l'une des revendications 1 à 3, **caractérisé en ce que** le côté intérieur (25) des branches (3, 4) sont dotés de nervures (30) qui s'étendent transversalement par rapport au cylindre de guidage (7).

5. Implant intervertébral selon l'une des revendications 1 à 4, **caractérisé en ce que** le côté extérieur (24) des branches (3, 4) est doté de nervures de guidage (12) qui s'étendent essentiellement en parallèle au cylindre de guidage (7).

6. Implant intervertébral selon l'une des revendications 1 à 4, **caractérisé en ce que** le côté extérieur (24) des branches (3, 4) est doté de nervures transversales qui s'étendent essentiellement en parallèle au cylindre de guidage (7) et en particulier d'un profil en arête de poisson ou un profil en dents de scie.

7. Implant intervertébral selon l'une des revendications 1 à 6, **caractérisé en ce que** les branches (3, 4) sont dotées de perforations (22).

8. Implant intervertébral selon l'une des revendications 1 à 7, **caractérisé en ce que** deux branches (3, 4) sont prévues dans chacun des deux plans (5, 6) du corps de soutien (1) en forme de U, les branches (3, 4) étant disposées aux extrémités de la branche centrale (2) de telle sorte qu'entre les deux branches (3, 4), on crée un espace libre (23) dans chaque plan (5, 6).

9. Implant intervertébral selon l'une des revendications 1 à 8, **caractérisé en ce que** la branche centrale (2) est disposée symétriquement par rapport aux branches (3, 4).

10. Implant intervertébral selon l'une des revendications 1 à 9, **caractérisé en ce que** sur leur extrémité avant libre, les branches (3; 4) présentent une lèvre (29) tournée vers l'intérieur.

11. Implant intervertébral selon l'une des revendications 1 à 10, **caractérisé en ce qu'**il comprend en outre une tige de guidage (31) qui peut être fixée dans le filet intérieur (8) du cylindre de guidage (7).

12. Implant intervertébral selon l'une des revendications 1 à 11, **caractérisé en ce qu'**il comprend en outre une douille (9) avec une partie (10) qui est dotée d'un filet extérieur et qui peut être passée sur le cylindre de guidage (7).

13. Implant intervertébral selon l'une des revendications 1 à 12, **caractérisé en ce qu'**il comprend en outre un dispositif d'écartement (11) pour les branches (3, 4) qui peut être inséré entre les plans (5, 6) et les branches (3, 4) qui y sont situées, le dispositif d'écartement présentant une vis (14) avec une tige (20) dotée d'un filet (15) qui correspond au filet intérieur (8).

14. Implant intervertébral selon l'une des revendications 1 à 12, **caractérisé en ce qu'**il comprend en outre un dispositif d'écartement (11) pour les branches (3, 4) qui peut être inséré entre les plans (5, 6) et les branches (3, 4) qui y sont situées, le dispositif d'écartement présentant un écrou cylindrique creux (26) doté d'un filet intérieur (27) qui correspond au filet extérieur (28).
